# EUROPEAN PATENT APPLICATION

(11) **EP 4 079 284 A1**
(43) Date of publication of application: **26.10.2022**
(21) Application number: 21382355.2
(22) Date of filing: 23.04.2021
(51) Int. Cl.: A61K 8/02, A61K 8/19, A61Q 19/06

(54) **A GRAPHENE NANOMATERIAL FOR USE AS A FAT ADBORBENT**

(71) Applicant: Graphenano Medical Care, S.L., 30510 Yecla (Murcia) (ES)
(72) Inventor: MARTÍNEZ ROVIRA, Martín, 30510 Yecla (Murcia) (ES); MARTÍNEZ ROVIRA, José Antonio, 30510 Yecla (Murcia) (ES); LAVÍN LÓPEZ, María del Prado, 30510 Yecla (Murcia) (ES); RODRÍGUEZ PUYOL, Diego, 28042 Madrid (ES); RODRÍGUEZ PUYOL, Manuel, 28042 Madrid (ES); DE FRUTOS GARCÍA, Sergio, 28821 Coslada. Madrid (ES); GRIEGA MARINO, Mercedes, 28805 Alcalá de Henares. Madrid (ES)
(74) Representative: Ibarra Garcia, Isabel

(57) **Abstract**

Provided is the use as dietary fat adsorbent in the digestive tract by oral administration of graphene-based nanomaterials, as the obtained from graphene nanofibers, graphene oxide, reduced graphene oxide or graphene nanotubes, having a modified crystal structure and a defined size distribution. The product is herein demonstrated to be non-toxic at enteric or systemic level after oral administration.

The product can be used to treat obesity and related complications and diseases. In fact the invention proves to reduce adipose tissue volume and weights distributed in the subcutaneous and visceral depots, to reduce hyperglycaemia, hypertriglyceridemia, hypercholesterolemia, and hepatic steatosis.

## Description

### FIELD OF THE INVENTION

The invention relates to new uses of graphene-based nanomaterials as food supplement, additive, or oral treatment for the reduction of dietary fat absorbance in the digestive tract, weight gain, overweight, obesity and related health complications such as hyperglycaemia, hypertriglyceridemia, hypercholesterolemia and the treatment of ectopic deposition of fat in the liver (known as hepatic steatosis).

The method using the compositions in vivo is additionally phyperrovided and includes effective reduction of enteric fat absorption, obesity-related weight gain, hyperglycaemia, hypertriglyceridemia, hypercholesterolemia, and hepatic steatosis. The oral treatment resulted safe, non-cytotoxic, without enteric inflammation or changes in systemic parameters.

### BACKGROUND OF THE INVENTION

The family of carbon and graphene-related nanomaterials includes graphene (a two-dimensional material made of sp2-hybridized carbon atoms arranged in a hexagonal honeycomb lattice), graphite, polycyclic aromatic hydrocarbons, carbon nanotubes, fullerenes, various graphene nanostructures of different dimensionalities (e.g., graphene nanofibers, graphene nanoparticles, graphene quantum dots, graphene nanoribbons, graphene nanomeshes, graphene nanodisks, graphene foams, graphene nanopillars), any combinations of other graphene-related materials, substituted graphene-related materials (e.g., the substitution of carbon atoms with N, B, P, S, Si, or others) and graphene-related materials (e.g. graphene oxide and reduced graphene oxide) functionalized with reactive functional groups (e.g., carboxyl groups, esters, amides, thiols, hydroxyl groups, diol groups, ketone groups, sulfonate groups, carbonyl groups, aryl groups, epoxy groups, phenol groups, phosphonic acids, amine groups, porphyrin, pyridine, polymers and combinations thereof [Lavin-Lopez MP et al. Physical Chemistry Chemical Physics, 2014, 16 (7): p. 2962-2970].

Recently the applicants of the present application filed the international filing applications no. WO2020/016319A1 and no. WO2020/016324A1 related to new carbon and graphene-based nanomaterials and their application in cosmetics and therapeutics.

The products featured in the above-mentioned patents have no toxicity, good biocompatibility and provided biological applications in therapy and cosmetics such as treatment on wounded skin, improvement of skin appearance and reducer of hypodermal fat volume, fatty lumps, cellulitis, and wrinkles. The starting carbon nanomaterial is any of the above mentioned carbon and graphene-related nanomaterials (e.g., graphene nanofibers, graphene oxide, reduced graphene oxide, graphene nanotubes, etc.)

In a preferred embodiment, the product of the invention and the examples for their applications featured in the above-mentioned patents is in the form of graphene nanofibers and named "GMC-1".

However, the mentioned patent applications did not disclose any use as a fat adsorbent and its advantages.

In this sense, the absorption of dietary fat, composed by triglycerides and cholesterol, is a key step in the development of overweight and obesity. Obesity contributes to the development of chronic diseases such as atherosclerosis, type 2 diabetes mellitus and the presence of steatosis in non-alcoholic fatty liver disease.

During the settlement of obesity, increased dietary triglycerides storage may increase inside the adipose tissue, producing fat depots weights gain. Furthermore, disbalanced storage and/or consumption of circulating blood metabolites glucose, triglycerides and cholesterol are determinant factors responsible for the obesity-related clinical conditions. Hyperglycaemia (defined as the increase in plasmatic glucose), hypercholesterolemia and hypertriglyceridemia (or hyperlipidemia, which is defined as the increase in plasma lipids including total cholesterol and triglycerides) are common dysmetabolic factors that determine metabolic disorders, such as atherosclerosis and arterial damage, which lead to cardiovascular complications, insulin resistance and ectopic presence of fat in sensible organs as the liver [Parhofer KG. Diabetes Metab J. 2015, 39(5):353-62]

Enterosorbents are oral agents with high adsorption at the gastrointestinal level (enterosorption) of exo- and endotoxins, pathogenic microorganisms, allergens or other organic components.

As enteric decontaminants, some enterosorbents are or may be used as hepatoprotective and nephroprotective [Ornillo C et al. Adv Chronic Kidney Dis. 2020 Jan;27(1):5-10, Juurlink DN et al. Br J Clin Pharmacol. 2016 Mar;81(3):482-7; Nikolaev VG et al Exp. Oncol. 2011, 33, 2-8; Sanaka T et al Am. J. Kidney Dis. 12, 97-103 1988; Owada, A. et al. Kidney Int. Suppl. 63, S188-S190, 1997; Aoyama I et al Kidney Int Suppl. 1999 Jul;71:S193-7; Nakada Y et al. Sci Rep. 2019 Oct 30;9(1):15571; Sakhno LA et al. , Exp Oncol. 2013 Mar;35(1):45-52; Shevchuk O et al. Medicina (Kaunas). 2019 Sep 2;55(9). pii: E557; Stedtfeld RD et al, Appl Microbiol Biotechnol. 2017 Oct;101(19):7409-7415].

In some cases, enterosorbents have been designed as lipid-lowering agents, to mitigate hyperlipidemia-related diseases by reducing the dietary lipids enteric absorption. [Howell CA et al. Sci Rep. 2019 Apr 4;9(1):5629; Krasopoulos JC et al. Lipids. 1980 May;15(5):365-70.; Milard M et al Mol Nutr Food Res. 2019 Feb;63(4):e1801078; Gutman R et J Med Food. 2020 Mar;23(3):289-296]-

Carbon and graphene-based nanomaterials feature high absorption capacities due to their unique physicochemical properties, such as a large accessible surface area, diverse surface and pore structures, high stability, and tuneable chemistry via functionalization that can modify the surface hydrophobicity. This alike-high adsorbent surface of these nanomaterials permits their extensive investigation in environmental applications or their addition during fat-based food manufacture to reduce fatty acid content, to name some examples of potential use [Kim S et al. Chemosphere. 2018 Dec;212:1104-1124]

Several publications describe the use of graphene materials for medical applications. However, there is still a need for new further uses of known graphene products as a fat adsorbent.

There is still a need for new graphene-based materials with low or no toxicity, good biocompatibility and able to provide a useful biological effect and applications in therapy.

### BRIEF DESCRIPTION OF THE INVENTION

The known products disclosed in the international filing applications no. WO2020/016319A1 and no. WO2020/016324A1 can be used in therapy and cosmetic in relation to the treatment of obesity and related diseases. The applicant has now found new uses of the known graphene products having remarkable properties. The present patent application provides a further use of these known graphene-based nanomaterials (e.g., GMC-1) as a dietary fat adsorbent in the enteric tract, and effective as dietary lipid-lowering agent. reducing fat absorbance and its presence in the circulating blood. Therefore, the oral administration of the products reduces the absorbance of fat by the intestine and the presence of fat in the circulating blood. Thus, the products use is intended to reduce overweight, obesity, and its related health complications.

The known graphene products used in the invention are selected from any of the above mentioned carbon and graphene-related nanomaterials, such as graphene nanofibers, graphene oxide, reduced graphene oxide, graphene nanotubes or combinations thereof, as stated in the above-mentioned patents.

The term "graphene" refers to a material forming a polycyclic aromatic molecule with multiple carbon atoms covalently bonded to each other. The covalently bonded carbon atoms form a six-member ring as a repeating unit.

The term "graphene oxide" may be abbreviated as "GO" and may include a structure in which a functional group containing oxygen such as a carboxyl group, a hydroxyl group, or an epoxy group is bonded onto graphene.

The term "reduced graphene oxide" refers to graphene oxide decreased in a percentage of oxygen through a reduction process and can be abbreviated as "RGO".

The term "Graphene nanofibers" (GNFs) refers to cylindric nanostructures with graphene layers arranged as stacked cones, cups or plates. The graphene plane surface is canted from the fibre axis, which exposes the plane edges present on the interior and exterior surfaces of the carbon nanofibers.

The term "graphene nanotubes" (GNTs) refers to single wall or multi-wall concentric cylinders of graphene where the basal planes form a less reactive surface compared to that of Graphene Nanofibers because they are cylindrical and hollow like a tube but graphene nanofibers are like rods and generally there is no inner empty space within them.

The starting carbon nanomaterial is a graphene-based material derived from GNF, GO, RGO or GNT synthesized following the methods reported in the above-mentioned patents. The raw graphene nanomaterial is then subjected to a purification process, treated to achieve a reduced particle size distribution, and finally an optional depyrogenization process, which makes the product suitable for medical and cosmetic uses.

GNF are especially preferred as starting material to prepare the product of the invention.

On this regard, the present invention relates to the aforementioned graphene nanomaterial for use as a fat adsorbent in oral administration wherein the graphene nanomaterial is selected from having a particle size distribution having a dn(90) of 0.60 µm or smaller in number of particle and a dv(90) of 80.00 µm or smaller in volume particle, as measured by laser diffraction particle analyzer.

Preferably, the BET (abbreviated from Brunner-Emmett-Teller) specific surface area of the graphene nanomaterial is comprised between 100 and 500 m2/g, more preferably between 300-350 m2/g, the pore volume is between 0.35-0.40 cm3/g, and the impurities are less than 0.01% by weight.

Concretely, the use of the graphene nanomaterials is directed to the treatment of obesity and related diseases by reducing adipose tissue weight gain in the abdominal/visceral cavity.

In another aspect, the invention is directed to the use of the above defined graphene nanomaterial in the treatment of obesity-related hyperglycaemia and hyperlipidemia.

In a further aspect, the invention is directed to the use of the above defined graphene nanomaterial in the treatment of diet induced hypercholesterolemia.

In a further aspect, the invention is directed to the use of the above defined graphene nanomaterial in the treatment of ectopic deposition of fat in the liver, reducing dyslipidemia and reducing hepatic steatosis.

The oral administration of the detailed products is provided as enteric orally ingestible compositions comprising the graphene product and one or more acceptable excipients.

In a preferred embodiment the composition of the graphene-based product is suitable for oral administration and may comprise conventional additives and adjuvants for oral applications.

Advantageously, the oral administration of the product of the present invention generates an improvement of the user in his treatment of overweight from a point of view of an aesthetical treatment. In this sense, it has been proven that the weight loss generates immediate aesthetic benefits, such as reducing clothing size, boosting self-confidence and improving mood.

Nevertheless, the intake of the present product could cause positive benefits in relation to the treatment of obesity and related diseases. In this sense, in case the user has overweight, that weight loss generates an improvement in health and fitness closely related to the reduction of adipose tissue volume in the visceral and subcutaneous cavities.

The daily dosage of the formulation comprising the graphene product of the invention may depend on various factors, such as sex, age, weight, and individual conditions. The amount of the graphene nanomaterial product in the oral formulation can be in the range of 0.05 % to 10% w/w, preferably from 0.05% to 0.5% w/w.

### FIGURES

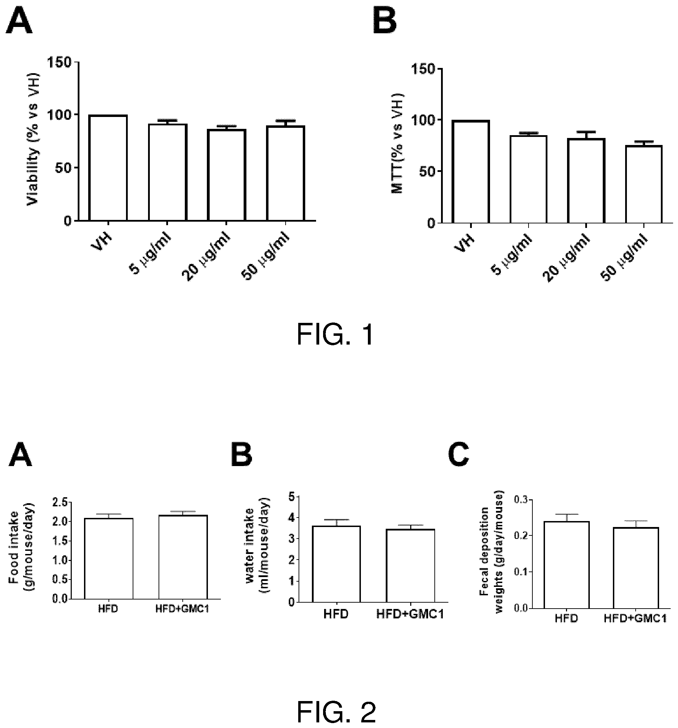

**Figure 1****.-** In vitro toxicity assays in cultured enteric human epithelial cells CACO-2 treated for 24 hours with graphene-based nanomaterial GMC-1 at different concentrations or vehicle (VH). A) Viability determined by trypan blue cellular exclusion and B) cellular metabolism determined by MTT ((3-(4,5-dimethylthiazol-2-yl)-2,5-diphenyltetrazoliumbromid)). The values are represented as mean +/- SEM.
**Figure 2****.-** In vivo safety analysis of food and water intakes and excretions during GMC-1 oral supplementation in mice subjected to HFD (High-fat diet). Animals housed in metabolic cages were challenged to HFD for 2 weeks and switched to HFD or HFD+GMC-1 for 4 weeks more. A) food intakes (g), B) water intake (ml) and C) faecal deposition weight (g) per animal and per day along the experimental period. The values are represented as mean +/-SEM.

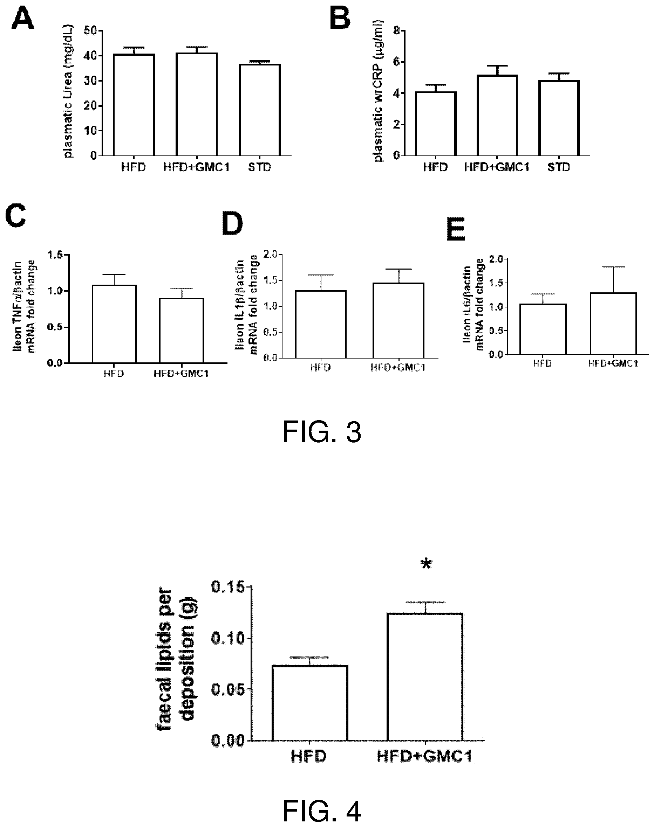

**Figure 3****.-** In vivo safety analysis during GMC-1 oral supplementation in mice subjected to HFD. Animals were challenged to HFD for 2 weeks and switched to HFD or HFD+GMC-1 for 4 weeks more. As control of HFD, other group of animals were subjected with STD (Low fat standard diet). Plasmatic values of A) urea B) and wrCRP (wide range C-reactive protein) were determined per animal and day in every diet challenged animal group along the period of experimentation. The 6th week of diets challenge, the mRNA expression fold change of enteric inflammatory markers C) TNF-alpha, D) IL-1beta and E) IL-6 were determined in extracted ileons by RT-qPCR (reverse transcription- quantitative polymerase chain reaction) using beta-actin expression as normalizing factor. The values are represented as mean +/-SEM.
**Figure 4****.-** Non-absorbed faecal lipids content during GMC-1 oral supplementation in mice subjected to HFD. Animals were challenged to HFD for 2 weeks and switched to HFD or HFD+GMC-1 for 4 weeks more. Faecal lipid extract weight (g) were normalized to the total deposition weight per animal. The values are represented as mean +/- SEM. * = p<0.05 vs HFD.

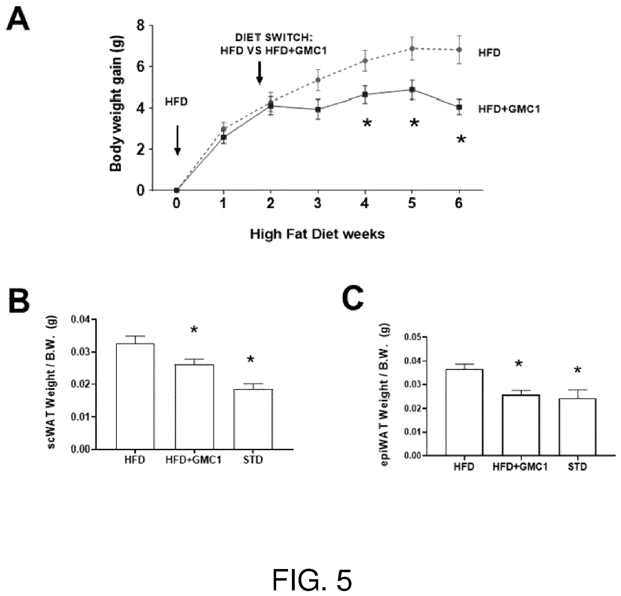

**Figure 5****.-** Body weight and adipose tissue depots during oral GMC-1 supplementation in mice subjected to HFD. Animals were challenged to HFD for 2 weeks and switched to HFD or HFD+GMC-1 for 4 weeks more. As control of HFD, other group of animals were subjected with STD. A) Body weight (b.w.) grams gain per week along the HFD or HFD+GMC-1 challenges. B) epididimal (epi) and C) subcutaneous (sc) white adipose tissue (WAT) depots weight ratios against total b.w. Values are represented as mean +/- SEM. *= P<0.05 vs. HFD.

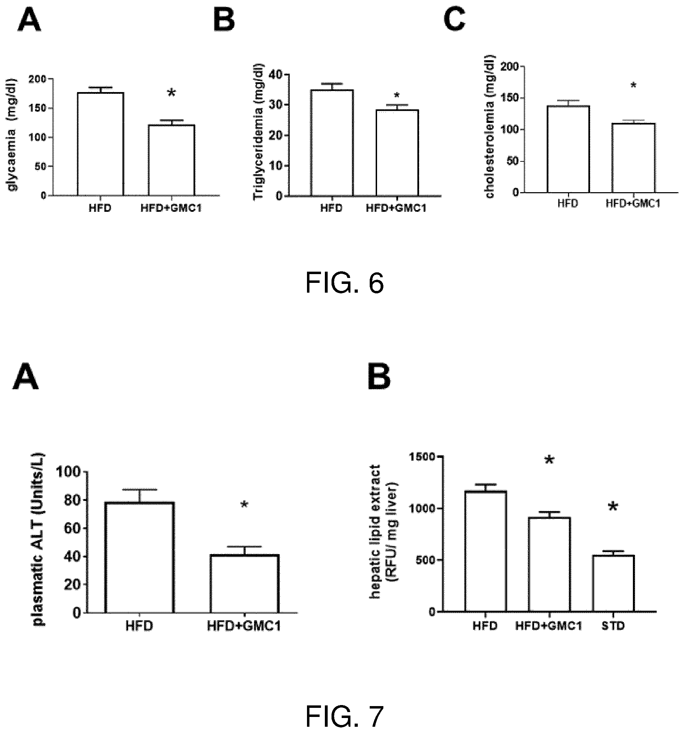

**Figure 6****.-** Blood levels of glucose, triglycerides and cholesterol during oral GMC-1 supplementation in mice subjected to HFD. Animals were challenged to HFD for 2 weeks and switched to HFD or HFD+GMC-1 for 4 weeks more. The 6th week of diets challenge, circulating levels of plasmatic A) glycaemia, B) triglyceridemia and C) cholesterolemia were determined. The values are represented as mean +/- SEM. *= P<0.05 vs. HFD.
**Figure 7****.-** Hepatic markers during oral GMC-1 supplementation in mice subjected to HFD. Animals were challenged to HFD for 2 weeks and switched to HFD or HFD+GMC-1 for 4 weeks more. As control of HFD, other group of animals were subjected with STD. The 6th week of diets challenge, A) circulating levels of plasmatic transaminase ALT was determined in HFD and HFD+GMC1. B) hepatic lipid extract weight (g) normalized to the hepatic weight sample were determined per animal in every diet challenged animal group. The values are represented as mean +/- SEM. * = p<0.05 vs HFD.

### DETAILED DESCRIPTION OF THE INVENTION

Herein we demonstrate in cultured cells and in an animal overweight and/or obesity model that the known graphene-based products disclosed in the international filing applications no. WO2020/016319A1 and no. WO2020/016324A1 (e.g., GMC-1) are safe and effective as lipid-lowering/enteric fat adsorbents. In a preferred embodiment, the product of the invention and the examples for their applications featured in the above-mentioned patents is in the form of graphene nanofibers and named "GMC-1".

As shown in the figures included in the present text, the graphene-based nanomaterial and compositions containing thereof resulted to be enteric-safe and useful as orally ingestible compositions in oral administration for non-therapeutic treatments (e.g., to enhance the fat distribution and the loss of body weight) as well as therapeutic in obesity and related diseases.

The term "therapy" or "therapeutic" is understood to mean treating or preventing disease in human beings or animals and restoring, correcting, or modifying physiological functions in humans or in animals.

All the data shown in figures are represented as the means ± SEMs of a variable number of experiments. Student's t test was used for 2 samples and 1- or 2-way analysis of variance (ANOVA) was used for >2 samples, with a paired or unpaired design followed by a multiple comparison test. Values of P < 0.05 were considered statistically significant.

Firstly, results of the biocompatibility essays of the product in vitro are depicted. Then, the following subsections depicted the results regarding the animal obesity model.

Advantageously, the analysis of the animal obesity model results allows us to conclude that the oral administration of the preferred embodiment of the graphene nanomaterial:
- Is safe and non-inflammatory after sub-chronical oral administration in vivo at the enteric level.
- Increases the presence of faecal lipids, therefore it is a fat adsorbent,
- Reduces visceral fat depots and so for the total body weight.
- Reduces the hyperglycaemia, hypertriglyceridemia and hypercholesterolemia
- Is useful for the treatment of ectopic deposition of fat in the liver.

### Biocompatibility of the product in vitro

A graphene-based biocompatible nanomaterial must be safe and non-toxic [Patel P et al. Curr Clin Pharmacol. 2017;12(2):73-82] To date the graphene-related toxicity is controversial in the literature, due to the diversity in several factors: productions conditions, defining characteristic of the material (e.g., shape, functionalization), concentration, time of exposure, administration or other conditions that affect the nanomaterial exposure to the biological sample [Rahmati M et al. Front Bioeng Biotechnol. 2019 Jan 23;7:4].

As evidenced by the examples shown on international patent applications no. WO2020/016319A1 and no. WO2020/016324A1, the products of the invention (e.g., GMC-1) are not toxic and have good biocompatibility in vitro. The examples shown in figure 1 demonstrate the innocuousness of GMC-1 in the context of oral administration of the product.

Biocompatibility and toxicological studies were achieved in cultured enteric human epithelial cells (Caco-2, ATCC HTB-37), a representative model of the first cellular barrier been in contact with the product during oral administration.

300,000 cells were seed per well in 6 wells culture dishes and grown with 10% fetal bovine serum supplemented culture media (by default, DMEM - dulbecco modified Eagles minimal essential medium-). The cells were maintained under controlled incubator conditions of 37ºC, 5% CO₂ and humidity. Once they reached confluence, they were deprived of serum for 16 hours. The product (GMC-1) was added in suspension at different concentrations over the cells, keeping the same final volume for 24 hours in the same controlled maintaining conditions. Afterwards, the cells were processed and were complementary tested for viability by Tripan blue exclusion and toxicity by cellular dyeing with MTT (3-(4,5-dimethylthiazol-2-yl)-2,5-diphenyltetrazoliumbromid).

Data are compiled and shown in figure 1. A range of GMC-1 (between 5 and 50 micrograms per ml of suspension) is non-toxic on cultured enteric epithelial cells. The data shows no difference in viability or cellular metabolism compared to control (vehicle-treated) cells.

### Animal obesity model

In the case of an excessively rich diet or for people not pursuing physical exercise, a substantial imbalance of the fat accumulated is established in the body, which includes increase of the body weight. The increase of the adipose tissue in the hypodermis may be gradually reflected by deformation of the skin brought about by the thickening of the hypodermis in which the subcutaneous adipose tissue is found, which can result in undesired skin appearance and cellulitis.

As explained in the introduction, obesity is considered a pathological overweight, where the excessive increase of adipose tissue volume and circulating metabolites are determinant in the development of chronic diseases such as atherosclerosis, type 2 diabetes mellitus and non-alcoholic fatty liver disease (NAFLD).

The effects of the oral administration of the product (eg. GMC-1) were tested in vivo, specifically on an animal (rodents) model of High-fat diet (HFD)-induced overweight/obesity, in order to test:
a) the enteric and systemic safety of the product during subchronical oral administration as a diet supplement,
b) the enteric fat absorption, and
c) the consequences in the body and fat pads weights, lipidemia, glycaemia and hepatic fat deposition.

HFD administration on rodents is a consistent model for early implementation of obesity along with early signs of related dysmetabolism [Williams LM et al PLoS One 2014 Aug 29;9(8):e106159; Guo S. J Endocrinol. 2014;220(2):T1-T23.].

Age-matched males and females mice (C57bl6 strain) were divided randomly into different groups and fed for a total 6 weeks as detailed below, with the following different diets:
- Low fat standard diet (STD, 13 kJ% fat, 67Kj% carbohydrates, 10 % sucrose; Envigo Teklad Global Diet 2014, East Millstone, NJ, USA).
- High Fat diet (HFD, 60 kJ% fat, 8.46 % sucrose; D12492 Ssniff Spezialdiäten, Soest, Germany).
- The same HFD supplemented with GMC-1 (0.5% W/W, HFD+GMC1), which corresponds to an ingestion of 625 mg GMC-1 per kg of body weight per day.

The functionally effective concentration used follows the state of the art published in similar approaches with other enteric adsorbents [Sakhno LA et al, Exp Oncol. 2013 Mar;35(1):45-52; Shevchuk O, Medicina (Kaunas). 2019 Sep 2;55(9). pii: E557; Howell CA, Sci Rep. 2019 Apr 4;9(1):5629. ; Stedtfeld RD, Appl Microbiol Biotechnol. 2017 Oct;101(19):7409-7415]

A total of 12 adult animals (3-5 months old) were subjected to HFD for 2 weeks. After 2 weeks under HFD, animals were divided randomly into 2 different groups (N=6 per group). The diet was switched to HFD+GMC-1 in one group and maintained with HFD in the other group. In some cases, a similar number of animals were subjected to STD, as basal conditions control. Diets were maintained for 4 additional weeks. All the diets were given to the animals "ad libitum" during the period of experimentation, with free access to water, under 12:12 h light:dark cycle and constant temperature of 21-23 °C. During or after the total period of diet challenges (total of 6 weeks), samples from the animals were collected, preserved and analyzed as described below.

Animal experiments were approved by the Institutional Animal Care and Use Committees in agreement with the guidelines established by the European Community Council Directives (2010/63/EU).

### Food intake and excretions during oral administration in vivo.

Diets are composed by different concentrations of carbohydrates, fats and other nutrients. As commented above, HFD and HFD+GMC-1 used in the experiments has 60% kJ of saturated fat. Plus, the latter is supplemented with the product.

To study whether the different diets may affect ingestion and excretion patterns, the above-mentioned animals were housed in metabolic cages to efficiently determine food and water intakes together with urine and faecal depositions per animal during the period of experimentation (6 weeks of diet challenge).

Figure 2 shows the mean values of intakes, urine volumes and faecal depositions per animal from the two different experimental groups HFD and HFD+GMC-1 during the experimental period. No difference in these patterns were observed between groups.

Thus, as it is shown in figure 2 the implementation of HFD with GMC-1 did not affected the disposition for the animal to food and water and did not changed the tract motility, periodicity or volumes of faecal depositions.

### Enteric and systemic safety tests in the animal model to determine the safety and biocompatibility of GMC-1 administered as a supplement in the diet.

At the end of the experimental period the animals were euthanized, and plasma and intestinal tissue were collected and preserved in order to study whether the continuous, sub-chronical oral administration of the product (4 weeks in HFD+GMC-1 group) was affecting the gastrointestinal integrity, enteric irritation and other biochemical parameters.

As mentioned above, a wide range of GMC-1 concentrations in a liquid suspension are potentially safe in cultured cells. To determine in vivo the safety and biocompatibility of GMC-1 administered as a supplement in the diet, blood parameters were determined using commercially available kits in extracted plasma along the period of experimentation from the same animals along their exposure to STD, HFD or HFD+GMC-1. The determined plasmatic parameters susceptible to be altered were urea as a renal failure marker (figure 3A) and the systemic inflammation marker wide range C-reactive protein (wrCRP) (figure 3B).

As mentioned above, the intestinal epithelium is the first barrier to be insulted by external oral toxicants. The response is based in the recruitment of the inflammatory cells to the mucosa, initiated by the epithelial chemokine production (e.g. TNF-alpha, IL-1beta, IL-6) [Castan L et al. Allergy. 2020 Feb;75(2):289-301; Sawa Y et al. Int J Mol Med. 2003 Feb;11 (2):175-9.]

Total messenger RNA (mRNA) from intestinal tissue samples was extracted and processed to reverse transcription- quantitative polymerase chain reaction (RT-qPCR) in order to study the expression fold changes between HFD vs HFD+GMC-1 of inflammatory markers (TNF-alpha, IL-1beta, IL-6). Briefly, 100 ng of converted DNA (cDNA) of the original mRNA extracted from the different samples were amplified with commercial gene expression assays (TaqMan, Life Technologies). The values were normalized against an endogenous control (beta-actin) and expression fold change was analyzed using the method 2^-deltadeltaCt.

The inflammatory markers were determined in extracted ileons and depicted also in figure 3: TNF-alpha (figure 3C), IL-1beta (figure 3D) and IL-6 (figure 3E). According to the observed non-difference for biochemical and other inflammatory markers between HFD and HFD+GMC-1, Figure 3 shows that GMC-1 is safe, non-inflammatory after sub-chronical oral administration in vivo at the enteric level.

### In vivo dietary fat enteric adsorption.

To study whether GMC-1 included in the diet was acting as enteric fat adsorbent, the determination of total faecal lipids in the animals depositions from HFD and HFD+GMC-1 groups were determined as a estimation of non-absorbed dietary fat during the diets challenge.

Thus, collected faecal samples from above-mentioned individual metabolic cages were analyzed for fat content using a well stablished lipid extraction and quantification protocol [Bligh EG and Dyer WJ. Can J Biochem Physiol 1959; 37: 911-917].

Per animal and faecal sample, the weight of dry fat extract was normalized to the total weight of the deposition. GMC1 impaired the dietary fat absorption, thus, increased the faecal fat presence, in the deposition of HFD+GMC1 mice when compared from those of HFD mice, as shown in figure 4.

Therefore, the values in figure 4 show that the inclusion of GMC-1 in HFD increase the presence of faecal lipids compared to HFD without including GMC-1.

### Body and adipose tissue depots weights in the animal model.

As commented above, overweight and obesity are defined as the increase of adipose tissue weight. Adipose tissue is distributed in different depots, from subcutaneous areas (e.g. subcutaneous white adipose tissue depot) to the abdominal/visceral cavity (e.g., in mice, epididymal white adipose tissue depot).

To study the weight changes as a consequence of the impaired fat absorption in animals subjected to HFD supplemented with GMC-1, the animals challenged to the different diets (either STD, HFD or HFD+GMC-1) were weighed periodically along the time of experimentation. After the period of experimentation (6 weeks), animals were euthanized, and fat depots were dissected and weighed.

Total body weights (B.W) and epididymal and subcutaneous fat depots weights (epiWAT and scWAT, respectively) per animal were recorded.

Figure 5 shows B.W (figure 5A), scWAT (figure 5B) and epiWAT (figure 5C) weight changes in HFD and HFD+GMC-1 along the weeks of experimentation. The depots weight values were normalized to B.W. at the end of the experimental procedure.

The ratios of each WAT depot against the weight of the same animal shows how much of the total B.W. change is due to fat mass weight change. Figure 5A, 5B and 5C depicted that GMC-1 supplementation with the diet reduced the gain of body and WAT depots weights during the 4 weeks of diet in HFD+GMC-1 compared to the gain in the same period observed in HFD mice.

The data collected proves that the total fat mass per animal was not increased during GMC-1 supplementation, as expected by the experimental model of HFD. This example demonstrates the capacity of the oral product to reduce visceral fat depots and so for the total body weight. Therefore, it is useful for the reduction of adipose tissue volume in the visceral and subcutaneous cavities.

### Dyslipidemia and hyperglycaemia in obesity model.

As commented above, clinical conditions that determine metabolic disorders (liver steatosis, atherosclerosis and insulin resistance) are related with the increase of circulatory glucose, triglycerides and cholesterol (hyperglycaemia and hyperlipidemia). At the end of the experimental period detailed above, the extracted plasmas were processed to determine circulating levels of glucose, total triglycerides and total cholesterol, using commercially available kits.

Figure 6 shows the reduction of the values of glycaemia (figure 6A), triglyceridemia (figure 6B) and cholesterolemia (figure 6C) with HFD+GMC-1 compared to the values from the HFD mice.

Therefore, the ingestion of GMC-1 in a HFD generates a reduction of the hyperglycaemia, hypertriglyceridemia and hypercholesterolemia in the animals as it is proved in the collected results in figure 6.

### Hepatic steatosis in obesity model.

As introduced above, the increased dyslipidemia induces hepatic steatosis, a pathological condition characteristic of NAFLD. Steatosis, which is the increased ectopic deposition of fat in the liver rather than the adipose tissue, lead to inappropriate liver function, and it can be biochemically measured in the circulating blood through the determination of increased levels of hepatic markers such as transaminases (e.g., alanine amino transferase, ALT).

At the end of the experimental period, plasma and livers were collected from the animals. The Fat content in the hepatic tissue was determined with a similar procedure as the above-mentioned faecal fat content analysis, with slight modifications [Bligh EG, Can J Biochem Physiol 1959].

Briefly, the total lipids were extracted and dried from a specific amount of hepatic tissue. The weight of the fat extract was normalized to the total weight of the liver portion. The biochemical determination of the circulating ALT was detected in the extracted plasma, using commercially available kits.

Figure 7 shows that circulating alanine amino transferase ALT (figure 7A) and hepatic fat content (figure 7B) were reduced HFD+GMC-1 compared to the values from the HFD mice. Therefore, the oral administration GMC-1 during HFD is useful in the treatment of steatosis or ectopic fat depositions in the liver.

To sum up, the performed essays allow us to prove that the graphene products of the invention disclosed in the international filing applications no. WO2020/016319A1 and no. WO2020/016324A1 are effective as fat adsorbents in oral administration.

Therefore, the graphene nanomaterial GMC-1 is useful in the treatment of obesity and related diseases because it is able to reduce the adipose tissue depots weight in the visceral and subcutaneal cavities, the hyperglycaemia, the hyperlipidemia, the hypercholesterolemia and other dyslipidemia related complications as the hepatic steatosis.

## Claims

1. A graphene nanomaterial for use as a fat adsorbent in oral administration wherein the graphene nanomaterial is selected from graphene nanofibers, graphene oxide, reduced graphene oxide and/or graphene nanotubes, and having a particle size distribution having a dn(90) of 0.60 µm or smaller in number of particle and a dv(90) of 80.00 µm or smaller in volume particle, as measured by laser diffraction particle analyzer.

2. A graphene nanomaterial for use according to claim 1, wherein its BET specific surface area is comprised between 100 and 500 m²/g.

3. A graphene nanomaterial for use according to claim 2, wherein its BET specific surface area is between 300-350 m²/g.

4. A graphene nanomaterial for use according to claim 1, wherein its pore volume is between 0.35-0.40 cm³/g.

5. A graphene nanomaterial for use according to claim 1, wherein its BET specific surface area is between 300-350 m²/g and its pore volume is between 0.35-0.40 cm³/g.

6. A graphene nanomaterial for use according to any claims 1 to 5, wherein the impurities in the nanomaterial are less than 0.01% by weight.

7. A graphene nanomaterial according to any claims 1 to 6, for use in the reduction of adipose tissue volume in the visceral and subcutaneous cavities.

8. A graphene nanomaterial according to any claims 1 to 6, for use in the treatment of obesity and related diseases.

9. A graphene nanomaterial according to any claims 1 to 6, for use in the aesthetical treatment of overweight.

10. A graphene nanomaterial according to any claims 1 to 6, for use in the treatment of hyperglycaemia.

11. A graphene nanomaterial according to any claims 1 to 6, for use in the treatment of hypertriglyceridemia.

12. A graphene nanomaterial according to any claims 1 to 6, for use in the treatment of hypercholesterolemia.

13. A graphene nanomaterial according to any claims 1 to 6, for use in the treatment of ectopic deposition of fat in the liver.
